# EUROPEAN PATENT APPLICATION

(11) **EP 4 516 310 A1**
(43) Date of publication of application: **05.03.2025**
(21) Application number: 23796535.5
(22) Date of filing: 28.04.2023
(51) Int. Cl.: A61K 38/48, A61P 31/04, C12N 9/36, C12N 15/63

(54) **BACTERIOLYTIC AGENT AGAINST ENTEROCOCCUS FAECALIS**

(30) Priority: 28.04.2022 JP 2022074458
(71) Applicant: The University of Tokyo, Bunkyo-ku, Tokyo 113-8654 (JP); University Public Corporation Osaka, Osaka-shi, Osaka 545-0051 (JP)
(72) Inventor: UEMATSU Satoshi, Tokyo 113-8654 (JP); FUJIMOTO Kosuke, Tokyo 113-8654 (JP); HAYASHI Tetsuya, Osaka-shi, Osaka 558-8585 (JP)
(74) Representative: Mewburn Ellis LLP
(86) International application number: PCT/JP2023/016830
(87) International publication number: WO 2023/210800

(57) **Abstract**

An object of the present invention is to provide a new lytic agent capable of lysing *Enterococcus* bacteria. Provided is a lytic agent for *Enterococcus* bacteria, consisting of a lytic enzyme or an active fragment thereof, the lytic enzyme comprising: (a) the amino acid sequence shown in SEQ ID NO: 1, (b) an amino acid sequence in which one or multiple amino acids are added, deleted, and/or substituted in the amino acid sequence shown in SEQ ID NO: 1, or (c) an amino acid sequence having a sequence identity of 90% or more with the amino acid sequence shown in SEQ ID NO: 1.

## Description

### Technical Field

The present invention relates to a lytic agent for *Enterococcus faecalis*, a pharmaceutical composition for treating or preventing an enterococcal infection, and the like.

### Background Art

Allogeneic hematopoietic cell transplantation (allogeneic transplantation) is a method in which a recipient is pretreated with *e.g.,* an anticancer agent, or irradiation and then undergoes transplantation of hematopoietic stem cells derived from a donor by *e.g.,* transfusion. Recipients who have undergone hematopoietic stem cell transplantation develop acute graft-versus-host disease (aGVHD), which is an intractable disease for 50% to 70% of the patients.

Acute graft-versus-host disease is a disease in which a donor-derived immune system introduced into the body of a recipient through allogeneic hematopoietic cell transplantation attacks and destroys tissues such as the skin, the digestive tract, or the liver. The mortality associated with acute graft-versus-host disease is reported to be 10 to 30%, and an effective treating or preventing method has not been established yet.

In recent years, it has been revealed that the development and exacerbation of acute graft-versus-host disease are closely associated with a change in the intestinal bacterial flora (Non-Patent Literatures 1 and 2). It has been reported that many examples have been recognized in which *Enterococcus* bacteria such as *Enterococcus faecalis* and *Enterococcus faecium* are dominant in the intestines of a patient with acute graft-versus-host disease, and that, in the intestinal bacterial flora, the higher the ratio of *Enterococcus* bacteria, the higher the mortality after allogeneic transplantation. Non-Patent Literature 3 discloses that the exacerbation of the intestinal bacterial flora disrupts the intestinal-tract barrier function in a recipient, inducing an abnormal immune response. Accordingly, a new treatment method targeting the intestinal bacterial flora has been expected to be developed as an effective method of treating acute graft-versus-host disease.

In the past, administration of an antibiotic has been attempted as a method of treating or preventing acute graft-versus-host disease targeting the intestinal bacterial flora. This method is intended to kill *Enterococcus* bacteria that have proliferated and become dominant in the intestines of a patient. However, the therapeutic efficacy of the method has not been observed (Non-Patent Literature 4). This is considered to be because administering an antibiotic can kill not only *Enterococcus* bacteria but also beneficial bacteria (good bacteria), and thus cannot restore the configuration of the intestinal bacterial flora to what it was before the development of a disease.

As another method targeting the intestinal bacterial flora, fecal microbiota transplantation (FMT) has also been attempted. The purpose of this method is to improve the intestinal bacterial flora by directly transplanting feces provided by a healthy person into the intestines of a patient. However, there are only a small number of reports for the efficacy of this method. In addition, there have also been a plurality of reports on a donor-derived drug-resistant-bacterial infection. Currently, the method has not been established as a safe standard treatment (Non-Patent Literatures 5 and 6).

Accordingly, a new method targeting the intestinal bacterial flora is necessary to treat and prevent acute graft-versus-host disease.

### Citation List

### Non-Patent Literature

Non-Patent Literature 1: Shono, Y., et al., Sci Transl Med., 2016, 8(339): 339ra71.
Non-Patent Literature 2: Gavriilaki, M., et al., Biol Blood Marrow Transplant, 2020, 26(9): 1738-46.
Non-Patent Literature 3: Ciernikova, S., et al., Blood Reviews, 2021, 48: 100790.
Non-Patent Literature 4: Gavriilaki, M., et al., Biol Blood Marrow Transplant, 2020, 26(9): 1738-46.
Non-Patent Literature 5: DeFilipp, Z. et al. N Engl J Med, 2019, 381: 2043-2050.
Non-Patent Literature 6: Bilinski, J. et al. Transpl Infect Dis, 2021, 23(1): e13386.

### Summary of Invention

### Technical Problem

An object of the present invention is to provide a new lytic agent capable of lysing *Enterococcus faecalis.*

### Solution to Problem

To solve the above-described problems, the present inventors have isolated an *Enterococcus faecalis* strain from a fecal sample which is derived from a patient who has undergone allogeneic hematopoietic cell transplantation, and in which *Enterococcus* bacteria has become dominant. Whole-genome analyses have been performed on a total of 11 isolates. As a result, a prophage sequence in the genome of the isolates and an endolysin gene in the prophage sequence have been found. It has been demonstrated that this phage-derived endolysin exhibits a remarkable lytic activity against *Enterococcus faecalis* isolates in an in vitro lysis test, and furthermore, has a surprising effect of dramatically enhancing the survival rate in the administration to model mice with graft-versus-host disease. A lytic enzyme of the present invention is extremely advantageous as compared to an antibiotic from the viewpoint of the capability of lysing *Enterococcus faecalis* selectively, and also has higher safety than fecal microbiota transplantation. The present invention is based on the above-described findings, and provides the following.

(1) A lytic agent for *Enterococcus faecalis*, consisting of a lytic enzyme comprising any of the amino acid sequences of the following (a) to (c) or an active fragment thereof:
   (a) the amino acid sequence shown in SEQ ID NO: 1;
   (b) an amino acid sequence in which one or multiple amino acids are added, deleted, and/or substituted in the amino acid sequence shown in SEQ ID NO: 1;
   (c) an amino acid sequence having 90% or more sequence identity with the amino acid sequence shown in SEQ ID NO: 1.
(2) A lytic agent for *Enterococcus faecalis*, comprising a polynucleotide encoding the lytic enzyme or an active fragment thereof of (1).
(3) The lytic agent of (2), wherein said polynucleotide comprises any of the base sequences of the following (a) to (d):
   (a) the base sequence shown in SEQ ID NO: 2;
   (b) a base sequence in which one or multiple bases are added, deleted, and/or substituted in the base sequence shown in SEQ ID NO: 2;
   (c) a base sequence having 90% or more sequence identity with the base sequence shown in SEQ ID NO: 2;
   (d) a base sequence which hybridizes with the base sequence complementary to the base sequence shown in SEQ ID NO: 2 under a high-stringent condition.
(4) A lytic agent for *Enterococcus faecalis*, comprising an expression vector comprising the polynucleotide of (2) or (3).
(5) A pharmaceutical composition for treating or preventing an enterococcal infection, comprising the lytic agent of any one of (1) to (4), and a pharmacologically acceptable carrier.
(6) The pharmaceutical composition of (5), wherein said enterococcal infection is intestinal dysbiosis, vancomycin-resistant enterococcus bacteremia, or infective endocarditis.
(7) The pharmaceutical composition of (6), wherein said intestinal dysbiosis is graft-versus-host disease or alcoholic liver disease.
(8) The pharmaceutical composition of any of (5) to (7), which is formulated as an enteric-coated preparation.
(9) The pharmaceutical composition of any of (5) to (8), wherein said carrier is any one or more selected from the group consisting of an excipient, binder, disintegrant, filler, emulsifier, plasticizer, and lubricant.
(10) The pharmaceutical composition of any of (5) to (9), which is for oral administration, rectal administration, intraperitoneal administration, or intravenous administration.

The contents of the disclosure in Japanese Patent Application No. 2022-074458 which forms the basis for priority of the present application are incorporated herein.

### Advantageous Effects of Invention

The present invention provides a new lytic agent capable of lysing *Enterococcus* bacteria.

### Brief Description of Drawing

[Figure 1] Figure 1 shows the genome of an *Enterococcus faecalis* isolate derived from a fecal sample provided by a patient who underwent allogeneic hematopoietic cell transplantation, and a prophage sequence contained in the genome. Figure 1A shows the genomic structure of an *Enterococcus faecalis* isolate (OCU031_14_8 strain). Figure 1B shows the structure of a prophage (OCU031_14_8 prophage) sequence.
[Figure 2] Figure 2 shows the results of detecting a synthesized phage-derived endolysin by SDS-PAGE.
[Figure 3] Figure 3 illustrates diagrams showing the results obtained by examining a lytic activity to an *Enterococcus faecalis* isolate (OCU031_14_8 strain). Figure 3A shows changes of OD₆₀₀ over time, assuming that the time point when the phage-derived endolysin was added to a cell resuspension liquid was 0 minute. Figure 3B shows the cell resuspension liquid one hour after the phage-derived endolysin was added.
[Figure 4] Figure 4 shows the results of a biofilm assay using crystal-violet staining. A vehicle (V) or a phage-derived endolysin (E) was added to total 11 *Enterococcus faecalis* isolates.
[Figure 5] Figure 5 shows the experimental method in Example 5 schematically.
[Figure 6] Figure 6 shows the results of administering a phage-derived endolysin to model mice with graft-versus-host disease (GVHD), to which an *Enterococcus faecalis* isolate had been administered. Figure 6A shows the results obtained by of measuring the amount of *Enterococcus faecalis* in feces. Figure 6B shows the survival rate of mice, assuming that the day (Day 0) when a hematopoietic stem cell was transplanted was the starting point.
[Figure 7] Figure 7 shows the experimental method in Example 6 schematically.
[Figure 8] Figure 8 shows the results of administering a phage-derived endolysin to model mice with graft-versus-host disease (GVHD), in which a fecal sample was transplanted. Figure 8A shows the ratio of *Enterococcus* bacteria in the feces. Figure 8B shows the survival rate of mice, assuming that the day (Day 0) when a hematopoietic stem cell was transplanted was the starting point.

### Description of Embodiments

### 1. Lytic agent for Enterococcus faecalis

### 1-1. Overview

A first aspect of the present invention is a lytic agent for *Enterococcus faecalis.* A lytic agent of the present invention consists of a lytic enzyme or an active fragment thereof, capable of lysing *Enterococcus faecalis*, or comprises: a polynucleotide encoding any of them; or an expression vector comprising the same.

### 1-2. Definitions of terms

The terms that are frequently used herein are defined below.

As used herein, a "lytic agent" refers to an agent consisting of a lytic enzyme having a lytic activity against target bacteria, or an agent comprising a polynucleotide encoding the lytic enzyme.

As used herein, the term "lysis" or "lytic" refers to a phenomenon of destruction of a bacterial cell membrane. Bacteria can be killed by lysis.

As used herein, a "lytic enzyme" refers to an enzyme having a lytic activity to *Enterococcus faecalis*, unless otherwise specified. Specifically, the lytic enzyme refers to any endolysin (wild-type endolysin) found in a prophage sequence of *Enterococcus faecalis*, and refers to a mutant endolysin derived from the endolysin, and having a lytic activity to *Enterococcus faecalis.*

As used herein, an "endolysin" refers to an enzyme derived from a bacteriophage, and having an activity of degradation of the cell wall of host bacteria. An endolysin usually has an activity of hydrolyzing peptidoglycan. A bacteriophage can exist as part of a bacterial genome or as an extrachromosomal plasmid without destroying a bacterial cell. This state is referred to as prophage. It is known that when a bacteriophage is released out of a bacterial cell after becoming a prophage or without becoming a prophage, an endolysin contributes to the release process by breaking the bacterial cell wall.

As used herein, an "enterococcus" is the generic term for indigenous bacteria having a spherical form found in the intestinal tract of an animal. An enterococcus is gram-positive facultative anaerobic bacteria, and 17 or more bacterial species are known as enterococci. Examples of enterococci include bacterial species such as *Enterococcus faecalis* and *Enterococcus faecium.*

As used herein, *"Enterococcus faecalis* (*E. faecalis*)" is one species of indigenous bacteria existing in the intestines of a human or an animal, and is a gram-positive group-D streptococcus. It is known that *Enterococcus faecalis* usually does not induce an infection in a healthy person, but induces various infections such as the below-described enterococcal infection, for example, in a person whose immunocompetence is decreased.

As used herein, an "enterococcal infection" is an infectious disease induced by infection by an enterococcus. Among enterococci, mainly *Enterococcus faecalis* and *Enterococcus faecium* induce an infection in a human. Unless otherwise specified, an enterococcal infection herein means an infection involved with *Enterococcus faecalis* infection, and refers to an enterococcal infection caused by *Enterococcus faecalis* infection in particular. Examples of the enterococcal infection caused by *Enterococcus faecalis* infection include, but are not limited to: intestinal dysbiosis such as (acute) graft-versus-host disease and alcoholic liver disease; vancomycin-resistant enterococcus bacteremia; and infective endocarditis.

As used herein, "graft-versus-host disease (GVHD)" is a disease caused by an attack that is made on an internal organ or tissue of a recipient by an immune system derived from a graft provided by a donor (*e.g.,* a bone marrow cell, peripheral blood, umbilical cord blood). Graft-versus-host disease is usually developed after the below-described allogeneic hematopoietic cell transplantation. Graft-versus-host disease is classified into: acute graft-versus-host disease which is developed acutely after transplantation; and chronic graft-versus-host disease which is developed after a certain period of time from transplantation. In this regard, the current classification is not based on the time of development, and in general, the two types are distinguished on the basis of characteristic observation.

As used herein, "acute graft-versus-host disease (aGVHD)" refers to a disease developed acutely after transplantation, among graft-versus-host diseases. Examples include a graft-versus-host disease that is developed within a given period of time (for example, within 100 days, typically within 6 days to 30 days). Examples of a symptom of acute graft-versus-host disease include: exanthems of and blisters of the skin; hepatic dysfunction (for example, jaundice); vomiting and hypophagia that are caused by the abnormality of the digestive tract; and persistent diarrhea.

As used herein, "chronic graft-versus-host disease (cGVHD)" refers to a disease developed after a given period of time from transplantation, among graft-versus-host diseases. Examples include a graft-versus-host disease that is developed 30 days to 100 days or more after transplantation. Compared with the above-described acute graft-versus-host disease, chronic graft-versus-host disease exhibits an autoimmune disease-like symptom, and, for example, shows the fibrillization of a tissue.

As used herein, "allogeneic hematopoietic cell transplantation" (often abbreviated as "allogeneic transplantation" or "allo-HCT") means hematopoietic stem cell transplantation from a donor other than the recipient. Usually, the donor is a blood relative or an unrelated person having the same or similar HLA type. Allogeneic hematopoietic cell transplantation is performed as a radical treatment of hematological malignancy such as leukemia, and classified into bone marrow transplantation, peripheral blood transplantation, or umbilical cord blood transplantation according to the species of the cells for use in the transplantation.

In the allogeneic hematopoietic cell transplantation, a conditioning regimen is usually performed. The "conditioning regimen" refers to a treatment to be performed to decrease the immunity of a recipient before allogeneic hematopoietic cell transplantation for the purpose of killing cancer cells and/or facilitating the engraftment of donor-derived hematopoietic stem cells. Specific examples of such a treatment include administration of an anticancer agent, irradiation, or a combination thereof; and, additionally, if desired, administration of an immunosuppressive agent before transplantation. Generally, the conditioning regimen is often performed approximately 7 to 10 days before hematopoietic stem cell transplantation.

As used herein, "alcoholic liver disease" refers to hepatic dysfunction induced by an excessive alcohol intake. Specific examples of hepatic dysfunction include alcoholic hepatic steatosis, hepatitis, and hepatic cirrhosis. It is known that alcoholic liver disease can be associated with *Enterococcus faecalis* infection (Duan, Y., et al., Nature, 2019, 575 (7783): 505-511).

As used herein, "vancomycin-resistant enterococcus bacteremia" refers to a pathological condition in which enterococci havig acquired resistance to vancomycin (a vancomycin-resistant enterococcus; VRE) is present in the blood flow. Usually, bacteremia does not particularly exhibit a symptom. However, a VRE proliferates in a specific tissue or internal organ, and thereby induces a serious infection in some cases. It is known that vancomycin-resistant enterococcus bacteremia can be associated with *Enterococcus faecalis* infection (Ford, C.D., et al., Biol Blood Marrow Transplant, 2017, 23 (2): 340-346).

As used herein, "infective endocarditis" refers to an infection caused in the heart. Infective endocarditis, with infection reaching a heart valve, induces valvular lesion or valvular heart disease in some cases. Infective endocarditis can occur if bacteria in the blood flow reach a heart valve having damage. It is known that infective endocarditis can be associated with *Enterococcus faecalis* infection (Ch'ng JH, et al., Nat Rev Microbiol, 2019, 17 (2): 82-94.).

As used herein, "dysbiosis" refers to an abnormal constitution of the bacterial flora in an organism. Specifically, dysbiosis means a state in which the constituent ratios of the bacterial species constituting the bacterial flora differ from that of a healthy person. It is known that dysbiosis involves the development or exacerbation of various diseases owing to a change in the constituent ratios of bacterial species, for example, a decrease in the number of bacterial species constituting the bacterial flora. In some cases, dysbiosis is accompanied by the killing of beneficial bacteria (good bacteria) and the inversion of the ratios of normal bacterial species (referred to as "microbial substitution"). As a result of dysbiosis, the bacterial flora has a bacterial constitution functionally poor as a whole. Herein, the dysbiosis of the intestinal bacterial flora is referred to as "intestinal dysbiosis" in particular. Examples of a disease accompanied by intestinal dysbiosis include, but are not limited to, graft-versus-host disease and alcoholic liver disease.

As used herein, the term "multiple" refers to, for example, 2 to 40, 2 to 30, 2 to 20, 2 to 15, 2 to 10, 2 to 7, 2 to 5, 2 to 4, or 2 to 3. In addition, an "amino acid identity" (a sequence identity related to an amino acid sequence) refers to the ratio (%) of the number of the amino acid residues identical in the amino acid sequence between two polypeptides in comparison, wherein the ratio is with respect to the number of all the amino acid residues, and wherein one or both of the amino acid sequences is/are aligned (set in alignment), if desired, with a gap(s) inserted suitably in the amino acid sequence(s) so that the number of the amino acid residues identical therebetween becomes the largest. To calculate an amino acid identity, two amino acid sequences can be aligned, using a known program such as Blast, FASTA, or ClustalW. A "base identity" (a sequence identity related to a base sequence) is calculated in the same manner.

As used herein, the "substitution (of an amino acid)" refers to a substitution in a group of conservative amino acids similar in properties such as an electric charge, side chain, polarity, and aromaticity among 20 kinds of amino acids that constitute natural proteins. Examples include a substitution in the following: the uncharged polar amino acid group having a low-polarity side chain (Gly, Asn, Gln, Ser, Thr, Cys, and Tyr); the branched-chain amino acid group (Leu, Val, and Ile); a neutral amino acid group (Gly, Ile, Val, Leu, Ala, Met, and Pro); a neutral amino acid group having a hydrophilic side chain (Asn, Gln, Thr, Ser, Tyr, and Cys); the acidic amino acid group (Asp and Glu); the basic amino acid group (Arg, Lys, and His); or the aromatic amino acid group (Phe, Tyr, and Trp). An amino acid substitution in these groups is known to be less prone to change the properties of a polypeptide, and thus, is preferable.

As used herein, a "stringent condition" means a condition under which a nonspecific hybrid is less prone to be formed. A "high-stringent condition" refers to a condition under which a nonspecific hybrid is less prone to be formed, or is not formed. Generally, a reaction condition with a lower salt concentration and a higher temperature is a higher-stringent condition. Such a condition is a condition under which washing after hybridization is performed with 0.1 × SSC and 0.1% SDS, for example, at 50°C to 70°C, 55°C to 68°C, or 65°C to 68°C. Besides, other conditions such as a probe concentration, a probe base length, and a hybridization time can be suitably combined to increase the stringency of hybridization.

### 1-3. Configuration

The configuration of a lytic agent of the present invention is specifically described below.

A lytic agent for *Enterococcus faecalis* according to the present invention (1) consists of a lytic enzyme or an active fragment thereof, (2) comprises a polynucleotide encoding a lytic enzyme or an active fragment thereof, or (3) comprises an expression vector comprising a polynucleotide encoding a lytic enzyme or an active fragment thereof, as an active ingredient.

### (1) Lytic enzyme or active fragment thereof

In one embodiment, a lytic agent of the present invention consists of, or comprises, a lytic enzyme or an active fragment thereof.

The lytic enzyme may be any one of an endolysin consisting of a wild-type amino acid sequence encoded by the prophage sequence of *Enterococcus faecalis* (hereinafter referred to as a "wild-type endolysin") and an endolysin consisting of a mutant amino acid sequence derived from the same endolysin (hereinafter referred to as a "mutant endolysin").

Examples of the wild-type endolysin include an endolysin consisting of the amino acid sequence shown in SEQ ID NO: 1.

The endolysin consisting of the amino acid sequence shown in SEQ ID NO: 1 (hereinafter referred to as a "phage-derived endolysin") is an endolysin found in common among the genomes of the 11 *Enterococcus faecalis* isolates isolated in Examples herein. All of the 11 isolates were found in the prophages classified into Podoviridae. This phage-derived endolysin comprises an enzymatic activity domain and a cell wall-binding domain. The enzymatic activity domain is a catalytic domain having an activity that catalyzes the hydrolysis of the 1,4β-bond between N-acetylmuramic acid and N-acetyl-D-glucosamine residue (an endo-N-acetylmuramidase activity or a muramidase activity). This enzymic activity can denature the bacterial cell wall. In the amino acid sequence shown in SEQ ID NO: 1, the enzymatic activity domain comprises position 3 to position 202. The cell wall-binding domain can be classified into ZoocinA_TRD domain, and comprises position 223 to position 329 in the amino acid sequence shown in SEQ ID NO: 1.

Examples of the mutant endolysin include a polypeptide comprising: an amino acid sequence in which one or multiple amino acids are deleted, substituted, or added in the amino acid sequence (the amino acid sequence shown in SEQ ID NO: 1) of the above-described wild-type endolysin; or an amino acid sequence having a sequence identity of 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more with the amino acid sequence (the amino acid sequence shown in SEQ ID NO: 1) of the above-described wild-type endolysin. The mutant endolysin preferably has an activity of 50% or more, 60% or more, 70% or more, 80% or more, or 90% or more of, or equal to or higher than, the activity of the wild-type endolysin. Examples of the mutant endolysin having such an activity include those comprising an enzymatic activity domain and a cell wall-binding domain. Examples include the mutant endolysin comprising an enzymatic activity domain consisting of position 3 to position 202 in the amino acid sequence shown in SEQ ID NO: 1 and a cell wall-binding domain consisting of position 223 to position 329 in the amino acid sequence shown in SEQ ID NO: 1.

In one embodiment, the lytic enzyme is a polypeptide or an active fragment thereof consisting of or comprising (a) the amino acid sequence shown in SEQ ID NO: 1, (b) an amino acid sequence in which one or multiple amino acids are added, deleted, and/or substituted in the amino acid sequence shown in SEQ ID NO: 1, or (c) an amino acid sequence having a sequence identity of 90% or more with the amino acid sequence shown in SEQ ID NO: 1.

As used herein, the "active fragment" of the lytic enzyme refers to a fragment that, in any of the above-described lytic enzymes, has a lytic activity to *Enterococcus faecalis*, for example, a fragment having an activity equal to 50% or more, 60% or more, 70% or more, 80% or more, or 90% or more of, or equal to or higher than, the activity of the above-described wild-type endolysin. Examples include a fragment comprising an enzymatic activity domain and/or a cell wall-binding domain. More specific examples include a fragment comprising an enzymatic activity domain consisting of position 3 to position 202 in the amino acid sequence shown in SEQ ID NO: 1 and/or a cell wall-binding domain consisting of position 223 to position 329 in the amino acid sequence shown in SEQ ID NO: 1. The amino acid length of the polypeptide constituting this fragment may be, for example, but not particularly limited to, a region of at least 50, 100, 150, 200, 250, or 300 consecutive amino acids in the wild-type endolysin.

### (2) Polynucleotide encoding a lytic enzyme or an active fragment

In one embodiment, a lytic agent of the present invention consists of or comprises a polynucleotide encoding a lytic enzyme or an active fragment thereof.

A polynucleotide according to the present invention encodes the above-described lytic enzyme or an active fragment thereof. A polynucleotide according to the present invention is not particularly limited to any base sequence as long as the polynucleotide encodes any of the above-described lytic enzymes or an active fragment thereof. Examples include a polynucleotide (for example, the polynucleotide consisting of the base sequence shown in SEQ ID NO: 2) encoding the wild-type endolysin consisting of the amino acid sequence shown in SEQ ID NO: 1.

In one embodiment, a polynucleotide according to the present invention comprises: (a) the base sequence shown in SEQ ID NO: 2; (b) a base sequence in which one or multiple bases are deleted, substituted, or added in the base sequence shown in SEQ ID NO: 2, (c) a base sequence having a sequence identity of 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more with the base sequence shown in SEQ ID NO: 2, or (d) a base sequence that hybridizes with the base sequence complementary to the base sequence shown in SEQ ID NO: 2 under a high-stringent condition.

In one embodiment, the base sequence of a polynucleotide according to the present invention may be a base sequence codon-optimized in accordance with the frequency of codon usage in the cell into which the polynucleotide is to be introduced.

A polynucleotide according to the present invention may be a DNA or an RNA such as an mRNA.

In a case where a polynucleotide according to the present invention is an mRNA, the base sequence thereof can be an mRNA comprising, as the coding region, a base sequence in which thymine (T) is substituted with uracil (U) in the above-described base sequences. An mRNA corresponding to a polynucleotide according to the present invention may comprise e.g., the cap structure of the 5' end, the poly A tail of the 3' end, the 5' untranslated region (5' UTR) upstream of the initiation codon, and/or the 3' untranslated region (3' UTR) downstream of the termination codon, in addition to said coding region. The 5' UTR, 3' UTR, and/or the like may comprise a sequence for regulating the amount of translation from the mRNA.

### (3) Expression vector comprising a polynucleotide encoding a lytic enzyme or an active fragment

In one embodiment, a lytic agent of the present invention comprises or consists of an expression vector comprising a polynucleotide encoding a lytic enzyme or an active fragment thereof, the lytic enzyme being capable of lysing *Enterococcus faecalis.*

An expression vector according to the present invention comprises a polynucleotide encoding a lytic enzyme of the present invention or a fragment thereof in a state that allows for the expression. As used herein, the "a state that allows for the expression" refers to a state in which a gene to be expressed is positioned in a region downstream of the promoter and under the control of the promoter.

An expression vector according to the present invention comprises, as essential constituents, a promoter and a polynucleotide described in "(2) Polynucleotide encoding a lytic enzyme or an active fragment".

A vector that can be used as an expression vector according to the present invention is, for example, an expression vector for which a plasmid or a virus is utilized. As used herein, an "expression vector" encompasses a plasmid vector, a virus vector, and a recombinant vector.

Examples of a promoter that can be used include various kinds of promoters such as overexpression promoters, constitutive promoters, site-specific promoters, stage-specific promoters, and/or inducible promoters. Examples include a CMV promoter (CMV-IE promoter), SV40 initial promoter, RSV promoter, HSV-TK promoter, EF1α promoter, Ub promoter, metallothionein promoter, SRα promoter, and CAG promoter.

The expression vector can comprise e.g., a terminator, enhancer, poly A signal, 5'-UTR (untranslated region) sequence, intron sequence, ribosome binding sequence, labeling- or selection-marker gene, multicloning site, nuclease recognition sequence, and/or origin of replication. Each of these species is not particularly limited, as long as it is capable of exerting its function in a host cell.

### 1-4. Effects

A lytic agent of the present invention can lyse *Enterococcus faecalis* selectively. A lytic agent of the present invention can lyse *Enterococcus faecalis* selectively, compared with bacteria other than *Enterococcus bacteria,* in the intestinal bacterial flora, and substantially does not lyse, for example, beneficial bacteria (good bacteria). Accordingly, the lytic agent can improve the state of the intestinal bacterial flora, without aggravating dysbiosis as an antibiotic does.

Accordingly to a lytic agent of the present invention, it possible to treat or prevent an enterococcal infection (for example, an enterococcal infection accompanied by dysbiosis) caused by *Enterococcus faecalis.*

A host cell into which an expression vector or a polynucleotide according to the present invention has been introduced is also provided.

Also provided is a method of treating or preventing an enterococcal infection, the method comprising a step of administering a lytic agent of the present invention to a subject, or a method of lysing *Enterococcus faecalis.* The enterococcal infection is, for example, acute graft-versus-host disease, and the subject may be a recipient of allogeneic hematopoietic cell transplantation.

Also provided is use of a lytic agent of the present invention in the production of a medicine for treating or preventing an enterococcal infection such as acute graft-versus-host disease.

### 2. Pharmaceutical composition for treating or preventing enterococcal infection

### 2-1. Overview

A second aspect of the present invention is a pharmaceutical composition for treating or preventing an enterococcal infection. A pharmaceutical composition for treating or preventing an enterococcal infection according to the present invention comprises a lytic agent for *Enterococcus faecalis* in the first aspect, and can treat or prevent an enterococcal infection.

### 2-2. Configuration

A pharmaceutical composition of the present invention comprises an active ingredient as an essential component, and in addition, a pharmacologically acceptable carrier or an additional drug as an optional component. A pharmaceutical composition of the present invention can be constituted by an active ingredient alone. However, to facilitate dosage formulation, and to maintain the pharmacological effect of the active ingredient and/or dosage form, the pharmaceutical composition is preferably constituted in the form of a pharmaceutical composition comprising the below-described pharmacologically acceptable carrier.

### 2-2-1. Components

Each component constituting a pharmaceutical composition of the present invention is described specifically.

### (1) Active ingredient

An active ingredient in a pharmaceutical composition of the present invention is a lytic agent of the present invention. The constitution of the lytic agent has already been described in detail in the first aspect, and here, the specific description of the constitution is omitted.

The number of lytic agents contained in a pharmaceutical composition of the present invention is not limited, and may be one or multiple. In a case where a pharmaceutical composition of the present invention comprises multiple lytic agents, the pharmaceutical composition can comprise any combination of the wild-type endolysin and/or the mutant endolysin described in the first aspect.

### (2) Pharmacologically acceptable carrier

A "pharmacologically acceptable carrier" refers to a solvent and/or an additive that can usually be used in the technical field of pharmaceutical formulation, and is substantially unharmful, or is not harmful at all, to an organism.

Examples of a pharmacologically acceptable solvent include water, ethanol, propylene glycol, ethoxylated isostearyl alcohol, polyoxygenated isostearyl alcohol, and polyoxyethylene sorbitan fatty acid esters. These are desirably sterilized, and preferably adjusted, if necessary, to be isotonic to blood.

In addition, examples of a pharmacologically acceptable additive include excipients, binders, disintegrants, fillers, emulsifiers, plasticizers, and lubricants.

Examples of the excipient include: sugars such as monosaccharides, disaccharides, cyclodextrins, and polysaccharides (more specific examples include, but are not limited to, glucose, sucrose, lactose, raffinose, mannitol, sorbitol, inositol, dextrin, maltodextrin, starch, and cellulose); metal salts (for example,, sodium chloride, sodium phosphate or calcium phosphate, calcium sulfate, magnesium sulfate, and calcium carbonate); citric acid; tartaric acid; glycine; low-, medium-, or high-molecular-weight polyethylene glycol (PEG); pluronic; kaolin; silicic acid; or combinations thereof.

Examples of the binder include: starch pastes produced using starch of corn, wheat, rice, or potato; simple syrup; glucose liquid; gelatin; tragacanth; methylcellulose; hydroxypropylmethylcellulose; carboxymethylcellulose sodium; shellac; and/or polyvinylpyrrolidone.

Examples of the disintegrant include said starches, lactose, carboxymethyl starch, crosslinked polyvinylpyrrolidone, agar, laminaran powder, sodium bicarbonate, calcium carbonate, alginic acid or sodium alginate, polyoxyethylene sorbitan fatty acid ester, sodium lauryl sulfate, monoglyceride stearate, or salts thereof.

Examples of the filler include said sugars and/or calcium phosphate (for example, tricalcium phosphate or calcium hydrogen phosphate).

Examples of the emulsifier include sorbitan fatty acid esters, glycerin fatty acid esters, sucrose fatty acid esters, and propylene glycol fatty acid esters.

Examples of the plasticizer and the lubricant include silicic acid salts, talc, stearic acid salts, and polyethylene glycol.

If desired, the pharmaceutical composition can comprise, in addition to the above-described additives, *e.g.,* a corrigent, dissolution aid (solubilizer), suspension, diluent, surfactant, stabilizer, absorption promoter (for example, quaternary ammonium salt or sodium lauryl sulfate), expander, humectant (for example, glycerin or starch), adsorbent (for example, starch, lactose, kaolin, bentonite, or colloidal silicic acid), disintegration inhibitor (for example, white sugar, stearin, cacao butter, or hydrogenated oil), coating agent, colorant, preservative, antioxidant, perfume, flavor, sweetener, and/or buffer.

### (3) Additional drug

A pharmaceutical composition of the present invention can comprise additional drug to the extent that the pharmacological effects of the above-described active ingredients are not lost. Here, examples of the "additional drug" include: drugs having a lytic activity to *Enterococcus faecalis* in the same manner as a lytic agent of the present invention; and other agents for treating an enterococcal infection. Examples include known therapeutic agents, an antibacterial agents or an intestinal regulator (for example, probiotics preparations). Specific examples of such a drug for graft-versus-host disease include a corticosteroid preparation, an immunosuppressant, a calcineurin inhibitor, a cyclophosphamide preparation, an antithymocyte globulin preparation, and a mesenchymal stem cell preparation. In addition, the drug may be a drug having a direct therapeutic effect on an enterococcal infection or a pharmacological effect unrelated to a lytic activity to *Enterococcus faecalis.* Examples include an agent for protecting gastric mucosa.

In a case where a pharmaceutical composition of the present invention is a drug combination comprising an additional drug, the pharmaceutical composition can be expected to have a synergistic effect such as the capability to inhibit an enterococcal infection multifacetedly, and thus, is convenient.

### 2-2-2. Dosage form

The dosage form of a pharmaceutical composition of the present invention is not particularly limited, as long as it does not, or is less prone to, inactivate a lytic agent of the present invention as an active ingredient, and can sufficiently produce a pharmacological effect *in vivo* after administration.

The dosage form can be classified into a liquid dosage form or a solid dosage form (including a semisolid dosage form such as gel) according to the form, and a pharmaceutical composition of the present invention may be in any one of the dosage forms. In addition, the dosage form can be roughly classified into an oral dosage form and a parenteral dosage form according to the method of administration, and, also in this respect, may be any one of these dosage forms.

Specific examples of the oral dosage form include: liquid dosage forms such as suspension, emulsion, and syrup; pulvis (examples of which include dusting powder, powder, and candy powder); and solid dosage forms such as granules, tablets, capsules, sublingual forms, and troche. In addition, examples of the parenteral dosage form include liquid dosage forms such as injections, suspensions, and emulsions; and solid dosages forms such as cream, ointment, plaster, wet packs, and suppositories. A preferable dosage form is any one of the oral dosage forms or, in the case of a parenteral dosage form, an injection of the liquid dosage form.

In one embodiment, a pharmaceutical composition of the present invention is formulated as an enteric-coated preparation. The enteric-coated preparation can be formulated as capsules, tablets, caplets, pills, troche, lozenge, pulvis, or granules. The coating of the enteric-coated preparation is not particularly limited, and an enteric-coated polymer known in the art may be used as the coating. For example, an enteric-coated polymer such as poly(methacrylic acid-co-methyl methacrylate) or EUDRAGIT (for example, EUDRAGIT L30 D-55) can be used.

2-2-3. Method of administration

To a pharmaceutical composition of the present invention, any method known in the art may be applied, as long as it can administer a lytic agent of the present invention as an active ingredient in an effective amount to an organism to treat or prevent an enterococcal infection.

As used herein, an "effective amount" refers to a necessary amount in which the active ingredient can exert its function, *i.e.,* a necessary amount in which an agent for treating or preventing according to the present invention can treat or prevent an enterococcal infection. The amount is also an amount that causes almost no harmful side effect or that causes no harmful side effect to an organism to which the agent is applied. This effective amount can vary, depending on the condition such as information on a subject, the route of administration, or the frequency of administration. The "subject" is an animal individual to which a pharmaceutical composition of the present invention is applied. A preferable subject is a human. The "information on a subject" refers to various information on an individual subject, and comprises, for example: the age, body weight, gender, whole-body health status, and drug sensitivity of a subject; and whether the subject is taking any or no medicine. The effective amount and a dose calculated on the basis thereof are determined according to the judgement of a doctor or a veterinarian, for example, in accordance with information on an individual subject. In a case where a pharmaceutical composition of the present invention needs to be administered in a large amount so as to produce a sufficient effect of treatment or prevention of an enterococcal infection, the pharmaceutical composition can be administered in several divided doses to alleviate a burden on a subject.

A method of administering a pharmaceutical composition of the present invention may be any of systemic administration or topical administration. Examples of the systemic administration include: intravascular injection such as intravenous injection; and oral administration. In addition, examples of the topical administration include rectal administration and intraperitoneal administration. A preferable method of administration is oral administration, rectal administration, intraperitoneal administration, or intravenous administration. An active ingredient in a pharmaceutical composition of the present invention is composed of a lytic agent of the present invention. Accordingly, in the case of oral administration, it is preferable to perform a suitable treatment, for example, using a suitable DDS (drug delivery system) to protect the active ingredient from being decomposed by a digestive enzyme.

In a case where a pharmaceutical composition of the present invention is administered or taken in, the dose or the intake amount is suitably selected in accordance with, *e.g.,* the age, body weight, symptom, and health status of a subject, and the type of the composition (e.g., a medicine, food and drink). For example, the dose or the intake may be 0.001 mg/kg/day to 1000 mg/kg/day, 0.01 mg/kg/day to 500 mg/kg/day, 0.1 mg/kg/day to 200 mg/kg/day, 1 mg/kg/day to 100 mg/kg/day, 5 mg/kg/day to 50 mg/kg/day, or 10 mg/kg/day.

### 2-2-4. Target disease

An enterococcal infection as a target of a pharmaceutical composition of the present invention is not particularly limited, as long as it is a disease caused by *Enterococcus faecalis* infection. Specific examples of the enterococcal infection include: intestinal dysbiosis (for example, acute graft-versus-host disease and alcoholic liver disease), vancomycin-resistant enterococcus bacteremia, and infective endocarditis. It is disclosed in the literature (Duan, Y., et al., Nature, 2019, 575 (7783): 505-511) that *Enterococcus faecalis* can be involved in alcoholic liver disease. It is disclosed in the literature (Ford, C.D., et al., Biol Blood Marrow Transplant, 2017, 23 (2): 340-346) that *Enterococcus faecalis* can be involved in vancomycin-resistant enterococcus bacteremia (infection).

In one embodiment, the enterococcal infection is graft-versus-host disease, preferably acute graft-versus-host disease. In a case where a target of a pharmaceutical composition of the present invention is (acute) graft-versus-host disease, a pharmaceutical composition of the present invention can be administered to a recipient of allogeneic hematopoietic cell transplantation. In this case, a pharmaceutical composition of the present invention can be administered before and/or after allogeneic hematopoietic cell transplantation. For example, the administration can be started 100 days, 90 days, 80 days, 70 days, 60 days, 50 days, 40 days, 30 days, 20 days, 10 days, 5 days, or 3 days before allogeneic hematopoietic cell transplantation, and/or can be administered up to 3 days, 5 days, 10 days, 20 days, 30 days, 40 days, 50 days, 60 days, 70 days, 80 days, 90 days, or 100 days after transplantation.

### 2-3. Effects

A pharmaceutical composition of the present invention can treat or prevent an enterococcal infection.

In a case where a pharmaceutical composition of the present invention is administered before development of acute graft-versus-host disease, selectively decreasing *Enterococcus faecalis* in the intestinal bacterial flora can prevent acute graft-versus-host disease. In a case where a pharmaceutical composition of the present invention is administered after development of acute graft-versus-host disease, selectively decreasing *Enterococcus faecalis* that has become dominant in the intestinal bacterial flora can improve the state of the intestinal bacterial flora. In addition, a pharmaceutical composition of the present invention may be administered in combination with a fecal microbiota transplantation therapy. For example, a pharmaceutical composition of the present invention may be administered simultaneously with, before, or after fecal microbiota transplantation.

### 3. Food composition for treating or preventing enterococcal infection

### 3-1. Overview

A third aspect of the present invention is a food composition for treating or preventing an enterococcal infection. A food composition for treating or preventing an enterococcal infection according to the present invention comprises a lytic agent for *Enterococcus faecalis* of the first aspect, and can treat or prevent an enterococcal infection. A food composition according to the present invention is taken in, eaten, or drunk, for example, as a functional food, food with function claims, food for specified health use, or functional nutritional food.

### 3-2. Configuration

### (Active ingredient of food composition)

An active ingredient in a food composition of the present invention is the above-described lytic agent for *Enterococcus faecalis.*

### (Food composition)

The "food composition" of the present invention encompasses, but is not particularly limited to, *e.g.,* food, drink, and functional food. A food composition according to the present invention is not particularly limited to any kind of food. Examples of the food include; processed cereals such as confectionery, noodles, bread, cooked rice, and biscuits; fish paste; dairy products; instant food; and seasoning. In addition, examples of the drink include tea drinks *(e.g.,* green tea, black tea, and oolong tea), fruit or vegetable drinks, oral rehydration solutions, carbonated drinks, soft drinks, nutrient drinks, and water.

A food composition of the present invention may be a functional food. A "functional food" in the present invention means a food having functionality for an organism. Examples include what is called health food in general, such as functional health foods, foods with function claims, special-purpose foods, nutritional supplementary foods, health supplementary foods, and supplements (for example, liquids, powders, tablets, or capsules), encompassing foods for specified health use and functional nutritional foods. A functional food of the present invention is preferably a supplement.

A functional food of the present invention may be *e.g.,* a solid preparation (*e.g.,* tablets, granules, powders, pills, or capsules), liquid preparation (*e.g.,* a liquid agent, suspension, or syrup), gel, or paste, or may be in the form of a usual food or drink (*e.g.,* a drink, or confectionery).

The amount of a lytic agent added to a food composition of the present invention is not particularly limited. Usually, the amount of the lytic agent added is 0.001 to 99% by weight, 0.01 to 10% by weight, or 0.1 to 10% by weight, for example, 90% by weight, 50% by weight, 10% by weight, 5% by weight, 3% by weight, 2% by weight, 1% by weight, 0.1% by weight, or 0.01% by weight, with respect to the total weight of the food.

A food composition of the present invention may further comprise an arbitrary food component. A food composition of the present invention may comprise *e.g.,* water, protein, carbohydrate, lipid, vitamin, mineral, amino acid, organic acid, organic base, juice, flavor, or prebiotics. In addition, a food composition in the present invention may comprise an additive such as a sweetener, perfume, or coloring agent.

### 3-3. Method of intake

The subject, frequency of intake, amount of intake, time of intake, target disease, and the like of a food composition of the present invention are in accordance with *e.g.,* the subject, frequency of administration, dose, timing of administration, and target disease in the "pharmaceutical composition for treating or preventing enterococcal infection" described above.

### 3-4. Effects

In a subject who has taken a food composition of the present invention, an enterococcal infection can be treated or prevented safely.

### Examples

### <Example 1: Analysis of intestinal bacterial flora in patients after allogeneic hematopoietic cell transplantation>

### (Purpose)

The composition of the enteric microbes of patients was analyzed for fecal samples provided by the patients who underwent allogeneic hematopoietic cell transplantation.

### (Method)

### (1) Collection of fecal samples

A total of 317 fecal samples were provided by 46 hematologic disease patients who underwent allogeneic hematopoietic cell transplantation (allo-HCT) in the Hematology & Hematopoietic Cell Transplantation Department, Osaka Metropolitan University Hospital, during the period from January 2019 to June 2020. In this regard, this research received ethical approval from the review committee in the facilities, and was performed with informed consent received from all the patients.

The collection of fecal samples from each patient was started before transplantation (within 14 days before the start of a conditioning regimen), and continued for 98 ± 3 days from the day (day 0) of transfusion of stem cells, or until the time of discharge from the hospital. In a case where a patient was not able to provide a fecal sample owing to the aggravation of the patient's whole-body status, the collection of a specimen was omitted.

### (2) Extraction of bacterial DNAs from fecal sample

Bacterial DNAs were extracted from fecal samples in accordance with a method described in the literature (Fujimoto K, et al., Cell Host Microbe, 2020, 28 (3): 380-9, e9). Specifically, the fecal sample stored in an RNA later (Invitrogen, Carlsbad, CA) was homogenized in 1 mL of an SM-plus buffer (100 mM NaCl, 50 mM Tris-HCl (the pH, 7.4), 8 mM MgSO₄·7H₂O, 5 mM CaCl·2H₂O, and 0.01% (w/v) gelatin), and then allowed to pass through a 100 µm cell strainer. The sample was incubated, at 37°C for 1 hour, with 1 mL of an SM-plus buffer comprising 20 mM EDTA, 100 µg/mL recombinant human lysozyme (Sigma Aldrich, St Louis, MO, USA), and 0.5 U/mL Achromopeptidase (Fujifilm Wako Pure Chemical Corporation, Tokyo, Japan). After the incubation, the supernatant was incubated with a 1/400-fold amount of 20 mg/mL proteinase K (Nacalai Tesque, Inc., Kyoto, Japan) and a 1/20-fold amount of 10% sodium dodecyl sulfate (SDS) at 55°C for 1 hour. Then, phenol/chloroform/isoamyl alcohol in an equal amount was added to the sample, and mixed. The resulting mixture was centrifuged at 16,000 × g for 5 minutes, and underwent chloroform extraction. The resulting extract was mixed with a 1/10-fold amount of 3 M sodium acetate and an equal amount of isopropanol, and then centrifuged at 16,000 ×g for 15 minutes. The supernatant was removed. Then, the pellets were washed with 70% ethanol, and air-dried. Then, the DNAs were resuspended in a 10 mM Tris-HCl buffer (the pH, 8.0).

### (3) 16S rRNA gene analysis

The V3-V4 region of 16S rRNA was PCR-amplified. PCR amplification at the first stage was performed for 20 cycles, using the forward primer (ACACGACGCTCTTCCGATCTCCTACGGGNGGCWGCAG, SEQ ID NO: 3) and the reverse primer (GACGTGTGCTCTTCCGATCTGACTACHVGGGTATCTAATCC, SEQ ID NO: 4). Then, PCR amplification at the second stage was performed for 8 cycles using a primer pair consisting of the overhang sequences (ACACGACGCTCTTCCGATCT, SEQ ID NO: 5; and GACGTGTGCTCTTCCGATCT, SEQ ID NO: 6) and an index sequence, and NEBNext multiplex Oligos for Illumina (Dual Index Primers Set 1, New England Biolabs Inc.). Subsequently, the amplification product was purified, using Agencourt AMpure beads. Using a MiSeq v3 Reagent kit and 15% PhiX spike (Illumina), sequencing was performed with a MiSeq instrument (Illumina, Inc., San Diego, CA). A 16S rRNA gene analysis was performed, using QIIME2.

### (Results)

On the basis of 16S ribosome RNA (rRNA) gene sequencing, analyses of the composition of the enteric microbes were performed for the fecal samples. As a result, dominance of *Enterococcus* was found in 89 fecal samples derived from 30 examples (65.2%). In this regard, dominance of *Enterococcus* means a state in which the constituent ratio of *Enterococcus* bacteria accounts for 25% or more of the whole bacteria comprised in the fecal sample.

### <Example 2: Analysis of Enterococcus bacterial strains isolated from fecal sample>

### (Purpose)

*Enterococcus* bacterial strains were isolated from the fecal samples in which dominance of *Enterococcus* was found in Example 1, and resistance to an antibacterial agent and gene expression were analyzed.

### (Methods and Results)

### (1) Identification of bacterial species

Each of the 30 fecal samples in which dominance of *Enterococcus* was found in Example 1 was cultured with an agar culture medium (a brain-heart infusion (BHI) culture medium supplemented with 20 µg/mL aztreonam, 20 µg/mL polymyxin B, 4 µg/mL amphotericin B, and 50 µg/mL triphenyltetrazolium chloride) for selecting *Enterococcus.* The samples were aerobically cultured at 37°C for 24 to 48 hours. Then, colonies were picked up, and further cultured in a BHI culture medium. Then, bacterial DNAs were extracted, using a PowerSoil DNA Isolation Kit (QIAGEN Sciences Inc., Germantown, MD, USA). The resulting bacterial DNAs were subjected to PCR amplification, using a primer pair (ATCAAGTAGTCT, SEQ ID NO: 7; and ACGATTCAAAGCTAACTG, SEQ ID NO: 8) for *E. faecalis* and a primer pair (TTGAGGCAGACCAGATTGACG, SEQ ID NO: 9; and TATGACAGCGACTCCGATTCC, SEQ ID NO: 10) for *E. faecium.* The resulting amplification products were analyzed, using MCE-202 MultiNA with DNA-12000, whereby the bacterial species were identified.

Among 30 strains, 11 strains were identified as *E. faecalis*, and 19 strains were identified as *E. faecium.*

### (2) Investigation of resistance to an antibacterial agent

The sensitivity to an antibacterial agent for each of the above-described 30 *Enterococcus* bacterial strains was invenstigated. Specifically, the bacterial strains prepared in 1 McFarland standard with physiological saline were diluted 500-fold with a BHI culture medium, and then aerobically cultured in solid-phase wells of an "Eiken" DP42 dry plate (Eiken Chemical Co., Ltd., Tokyo, Japan) for 24 hours.

The minimal inhibitory concentration of any one of the antibacterial agents, *i.e.,* daptomycin (DAP), vancomycin (VCM), teicoplanin (TEIC), and linezolid (LZD), was determined in accordance with the instruction of the product. All of *E. faecalis* strains (11 strains) and *E. faecium* (19 strains) exhibited sensitivity to daptomycin, vancomycin, teicoplanin, and linezolid. The results obtained by testing resistance to the antibacterial agents of the *E*. *faecalis* strains (11 strains) are shown in Table 1 below.

**[Table 1]**

| Table 1: Resistance of E. faecalis isolates to antibacterial agents | | | | |
|---|---|---|---|---|
| *E. faecalis* strain | Minimal inhibitory concentration (µg/mL) | | | |
| | DAP | VCM | TEIC | LZD |
| OCU009_35_4 | >4 | 2 | 1 | 2 |
| OCU009_35_10 | >4 | 4 | 2 | 2 |
| OCU015_56_6 | >4 | 2 | 0.5 | 2 |
| OCU015_56_7 | >4 | 2 | 0.5 | 2 |
| OCU031_pre_6 | >4 | 2 | 1 | 2 |
| OCU031_pre_7 | >4 | 2 | 1 | 2 |
| OCU031_pre_10 | >4 | 2 | 1 | 2 |
| OCU031_7_1 | >4 | 4 | 2 | 2 |
| OCU031_7_9 | >4 | 2 | 2 | 2 |
| OCU031_14_5 | >4 | 4 | 1 | 2 |
| OCU031_14_8 | >4 | 2 | 1 | 2 |

| | | | | |
|---|---|---|---|---|
| * DAP, daptomycin; VCM, vancomycin; TEIC, teicoplanin; LZD, linezolid. | | | | |

It is reported that multidrug-resistant bacteria such as vancomycin-resistant *Enterococcus* bacteria (VRE) are associated with the mortality after allo-HCT (Ford, C.D., et al., Biol Blood Marrow Transplant, 2017, 23 (2): 340-6.). However, the above-described results agreed with the standard epidemiologic characteristics of *E. faecalis* strains and *E*. *faecium* strains isolated in Japan (Fujiya, K., et al., Sci Rep., 2021; 11 (1): 14780.).

### (3) Identification and gene expression analysis of cytolysin-related gene

Cytolysin in humans and many animal models is known as one of the exocrine proteins that contribute to exacerbation of an *Enterococcus-bacteria-related* disease. Using PCR, cytolysin-related genes (cylL_{L}, cylA, cylB, and cylM) were detected in all the *E. faecalis* isolates, but were not detected in *E. faecium* isolates.

Next, *E. faecalis* isolates and *E. faecium* isolates were analyzed for gene expression. Specifically, bacterial DNAs were extracted from isolated *E. faecalis* bacterial strains and *E. faecium* bacterial strains, and a sequence library was prepared. Using the library prepared, the whole-genome sequencing was performed with a next-generation sequencer. A contig was prepared by connecting the sequence reads obtained. Gene regions in the contig were identified, and checked with the database to search for functional genes. As a result, K01791, K07173, K05946, K08605, and the like were detected with high frequency in *E. faecalis* as Kyoto Encyclopedia genes and genomes (KEGG) Ontology (KO) terms related to biofilm formation. This result suggests that the survival and proliferation of *E. faecalis* in the intestines are based on biofilm formation.

### <Example 3: Whole-genome analysis of E. faecalis isolates, and identification of phage-derived endolysin>

### (Purpose)

*E. faecalis* isolates were used as a subject of whole-genome analysis. Furthermore, the lytic enzyme gene was identified in the prophage sequence in the genome of *E. faecalis.*

### (Methods and Results)

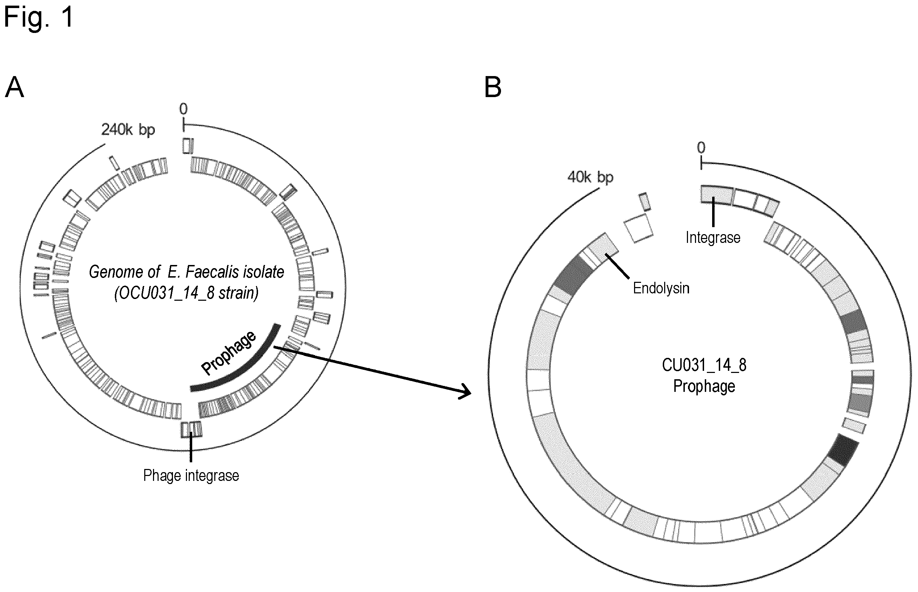

The shotgun sequence data of *E. faecalis* and *E. faecium* were analyzed, and prophage sequences were extracted. Specifically, a DNA library was prepared using a KAPA HyperPlus Kit (KAPA Biosystems, Inc., Indianapolis, IN, USA) in accordance with the attached manual except that NEBNext Multiplex Oligos (New England BioLabs, Ipswich, MA, USA) was used in the steps of adapter ligation and barcoding. The library was pooled, and sequencing was performed with a HiSeq2500 sequencer (2 × 250 paired end reads, HiSeq Rapid SBS Kit v2 (Illumina, Inc., San Diego, CA, USA)). The open reading frame (ORF) identified as a result of the sequencing of each sample underwent annotation in accordance with the KEGG prokaryotic gene and the corresponding KO (Figure 1A). In addition, the prophage sequence in the bacteria contig was predicted with VirSorter (v1.0.3) (Figure 1B). From the sequence data of each isolate, a maximum of four kinds of prophage sequences classified into Siphoviridae or Podoviridae were identified.

From 11 *E. faecalis* isolates, putative encoding sequences of endolysin were extracted from the prophage sequences. All of the endolysins identified consisted of the amino acid sequence shown in SEQ ID NO: 1, and encoded by the base sequence shown in SEQ ID NO: 2. Hereinafter, the endolysin found in common in the isolates is referred to as a "phage-derived endolysin", and the gene encoding the endolysin is referred to as a "phage-derived endolysin gene".

### <Example 4: Verification of lytic activity against E. faecalis isolate>

### (Purpose)

The phage-derived endolysin identified in Example 3 was synthesized, and an *in vitro* lytic activity for *E. faecalis* isolate was investigated.

### (Methods and Results)

### (1) Protein synthesis

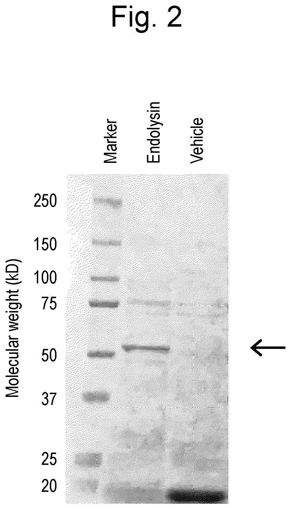

Using the genomic DNA of the *E. faecalis* isolate OCU_009_35_4 as a template, and using the forward primer (AAGGATCCATGACATTAAACGGAATTGA, SEQ ID NO: 11) and the reverse primer (AAGTCGACTTACCCATAGATTAATTTCT, SEQ ID NO: 12), a phage-derived endolysin gene was PCR-amplified, and the resulting amplified product was ligated to the BamHI/SalI site of a pCold-SUMO expression vector (Creative Biogene, Inc., Shirley, NY) to produce a His-SUMO-labeled endolysin expression vector. This expression vector was transformed into a BL21 (DE3) cell, and a protein of interest was purified using a Capturem (trademark) His-tag-purified maxiprep column (Takara Bio Inc.). The protein obtained was desalted with a HiTrap buffer (50 mM Na₃PO₄; 0.15 M NaCl; the pH, 7.0) through a HiTrap (trademark) desalting column (Amersham Biosciences Corp.). The protein solution was loaded into Amicon (registered trademark) Ultra-15 10K (Millipore), and centrifuged at 5,000 × g at room temperature for 20 minutes. The concentration of the target protein was measured, using Protein Assay CBB Solution (Nacalai Tesque, Inc.). The phage-derived endolysin obtained was confirmed by SDS polyacrylamide gel electrophoresis (SDS-PAGE) (Figure 2).

### (2) Lytic activity against E. faecalis isolate OCU031_14_8

The *E. faecalis* isolate OCU031_14_8 was aerobically cultured in a BHI culture medium, and centrifuged at 3,000 × g for 15 minutes for collection. The cell pellets were washed, and resuspended in a HiTrap buffer. The phage-derived endolysin was added at a final concentration of 50 µg/mL to the cell resuspension liquid. The turbidity (OD₆₀₀) was measured with a TVS062CA BioPhoto recorder (ADVANTEC Co., Ltd., Tokyo, Japan) every 1 minute to measure the lytic activity.

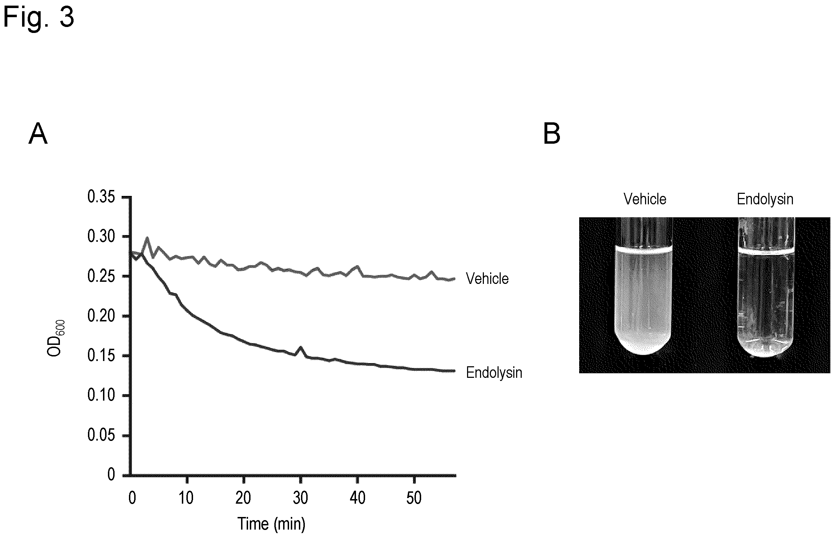

The results are shown in Figure 3. It was demonstrated that the *E. faecalis* isolate OCU031_14_8 was lysed by adding the phage-derived endolysin.

### (3) Lytic activity against 11 species of E. faecalis isolates

A biofilm assay was performed, using crystal-violet staining, in accordance with a method described in the past literature (Yang, Y., et al., J Appl Oral Sci., 2018, 26: e20170566; Shao, C., et al., J Proteome res., 2012, 11 (9): 4465-75; He Z., et al., Int J Antimicrob Agents., 2012, 40 (1): 30-5). This assay is a method in which a crystal violet pigment is adsorbed into a bacteria-derived biofilm (comprising an extracellular matrix and the like), and then, ethanol is added to the resulting biofilm to elute the crystal violet pigment, the absorbance of which is measured to quantify the amount of the pigment. The amount of the crystal violet pigment adsorbed shows first order correlation with the dry weight of the biofilm, and thus, this method can quantitatively evaluate the amount of the bacteria-derived biofilm.

Specifically, each of the 11 species of *E*. *faecalis* isolates was cultured at 37°C overnight in a BHI culture medium supplemented with aztreonam (20 µg/mL), polymyxinB (20 µg/mL), and amphotericinB (4 µg/mL). Then, the isolate was diluted 100-fold with a new BHI culture medium, inoculated into a 96-well flat-bottomed polystyrene microtiter plate (Corning, Inc., Arizona, USA, 353072), and aerobically cultured further at 37°C for 24 hours. Then, the 96-well plate was washed once with 200 µL of a HiTrap buffer, and 200 µL of phage-derived endolysin or a vehicle (SUMO protein having a His tag added thereto) was added (at a final concentration of 50 µg/mL). The plate was incubated overnight, then washed with 200 µL of phosphate buffered saline (PBS), turned upside down, and dried. Then, 200 µL of a 0.5% crystal violet solution was added to stain the isolate. The isolate was stained at room temperature for 15 minutes, and then, the plate was washed three times with 200 µL of PBS. The plate was dried at 50°C for 30 minutes, and 200 µL of 95% ethanol (v/v) was added to the pigment bound, which was thus dissolved. The absorbance of the sample was measured at 570 nm. For all the 11 species of bacterial strains, the experiment was repeated in 8 wells.

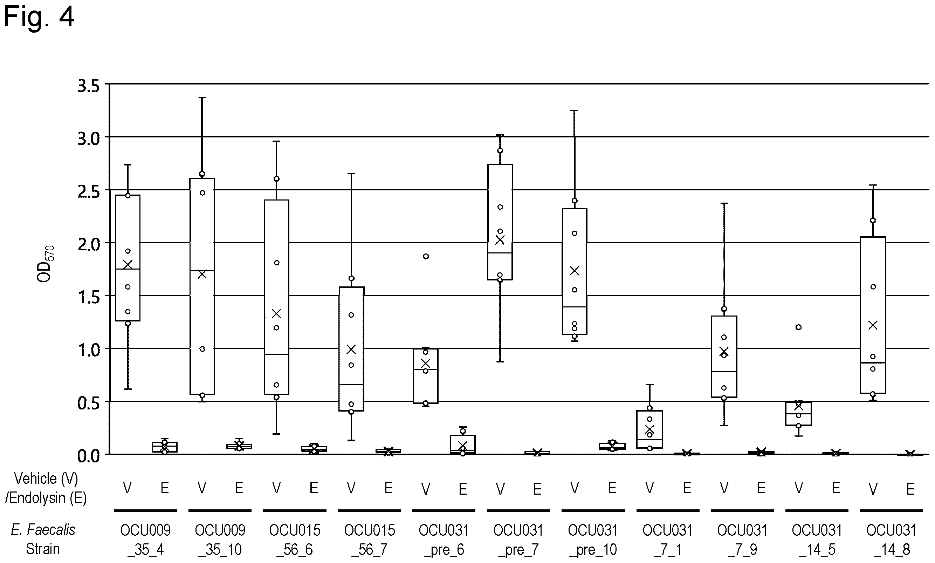

The results are shown in Figure 4. This result demonstrated that the biofilms of all 11 species of *E. faecalis* strains were lysed by the phage-derived endolysin.

### <Example 5: Administration of the phage-derived endolysin to the GVHD model mice to which E. faecalis was administered>

### (Purpose)

To the GVHD model mice to which *E. faecalis* was administered, the phage-derived endolysin was administered, and the influence on the number and survival rate of the *E. faecalis* bacteria in feces were studied.

### (Method)

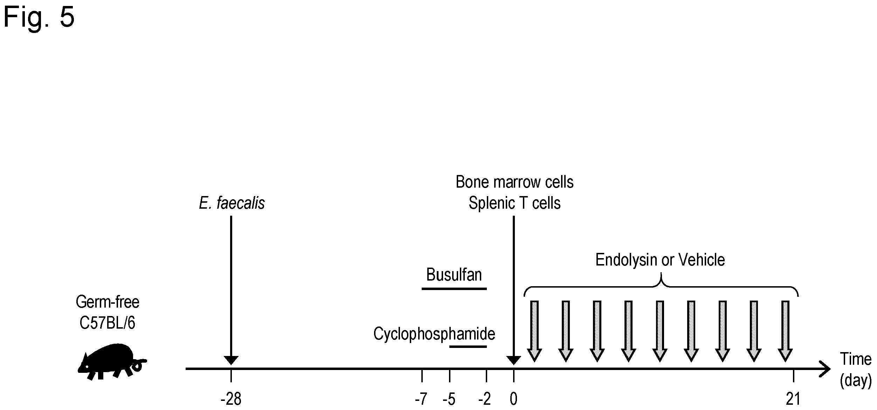

All the animal experiments were performed with the approval of the Animal Care and Use Committee of Osaka Metropolitan University. Germ-free C57BL/6 female mice (H2k^{b}) and SPF 129SvJ/JmsSlc female mice (6 to 8 weeks old) were purchased from SLC Japan Inc. (Hamamatsu, Japan) and CLEA Japan, Inc. (Tokyo, Japan) respectively. The mice were reared under temperature control (23 ± 2°C) under dark-period conditions from 8 pm to 8 am, and allowed to freely take sterile water and standard solid food for laboratory mice. The germ-free C57BL/6 mice were reared, one individual in one cage. The overview of the experimental method in the present Example is shown in Figure 5.

Before the stem cell transplantation, a suspension of *E. faecalis* OCU031_14_8 strain was orally administered to the germ-free C57BL/6 female mice. The suspension used was a suspension in which a 2.0×10⁸ colony forming unit (cfu) was suspended in 200 µL of sterile PBS. Three weeks later, a feces suspension was cultured with an agar culture medium (a brain-heart infusion (BHI) culture medium supplemented with 20 µg/mL of aztreonam, 20 µg/mL of polymyxin B, 4 µg/mL of amphotericin B, and 50 µg/mL of triphenyltetrazolium chloride) for selecting *Enterococcus,* whereby the colonization of *E. faecalis* was confirmed.

In a conditioning regimen, busulfan (Sigma-Aldrich) was intraperitoneally administered to the germ-free C57BL/6 female mice at 20 mg/kg/day for 5 days, and then cyclophosphamide (Sigma-Aldrich) was administered to the mice at 10 mg/kg/day for 3 days. Day 2 and Day 1 were rest days. On Day 0, 1.5×10⁷ bone marrow cells and 2.0×10⁶ spleen T cells, derived from 129SvJ/JmsSlc donor female mice, were intravenously injected into the recipient C57BL/6 mice. The bone marrow cells were obtained from a thighbone, and suspended in a Hanks' Balanced Salt Solution (Nacalai Tesque, Inc.). The red blood cells were lysed, using a red blood cell lysing buffer Hybti-Max (Sigma-Aldrich, RNBG8098), and then, a single cell suspension was prepared in PBS. To prepare a spleen T cell suspension, a Pan T cell isolation kit II (Miltenyi Biotec B.V. & Co. KG, Bergisch Gladbach, Germany, #130-095-130) for mice was used.

From the day (Day 0) of stem cell transplantation, 200 µg of the phage-derived endolysin or a vehicle (SUMO protein having a His tag added thereto) dissolved in 200 µL of a HiTrap buffer was orally administered three times a week (10 mg/kg/day, 30 mg/kg/week) until the third week.

The survival rate after Day 0 was monitored every day. All the survival periods of the groups were statistically analyzed and compared using a generalized Wilcoxon test. In addition, a suspension having feces diluted with a BHI culture medium was cultured in an agar culture medium for selecting *Enterococcus.* The number of colonies formed was measured to quantify the amount of *E. faecalis* bacteria in the feces. All the experiments were each performed as two repetitive experiments.

### (Results)

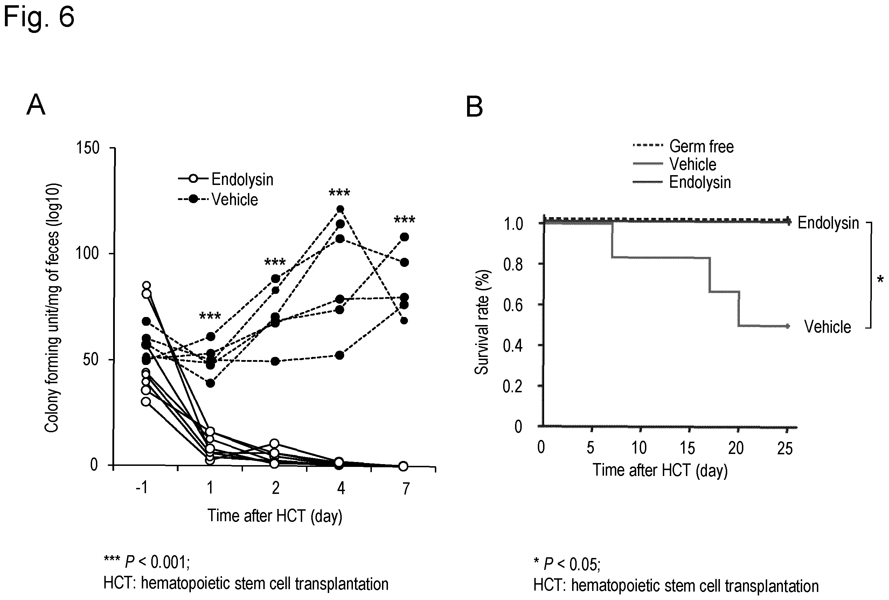

The results are shown in Figure 6. The GVHD model mice to which a vehicle (SUMO protein having a His tag added thereto) was administered exhibited a tendency to increase in the amount of the *E. faecalis* bacteria in the feces. On the other hand, the GVHD model mice to which the phage-derived endolysin was administered exhibited a noticeable decrease in the amount of the *E. faecalis* bacteria in the feces (Figure 6A).

In addition, half of the GVHD model mice to which the vehicle was administered died 25 days after the stem cell transplantation. Compared with this, 100% of the GVHD model mice to which the phage-derived endolysin was administered survived (Figure 6B). Accordingly, it was demonstrated that the administration of the phage-derived endolysin markedly improves the survival rate.

### <Example 6: Administration of the phage-derived endolysin to the GVHD model mice in which fecal sample was transplanted>

### (Purpose)

In the GVHD model mice to which the feces collected from a patient in Example 1 had been administered, the phage-derived endolysin was administered, and the influence on the number and survival rate of the *E. faecalis* bacteria in feces was studied.

### (Methods and Results)

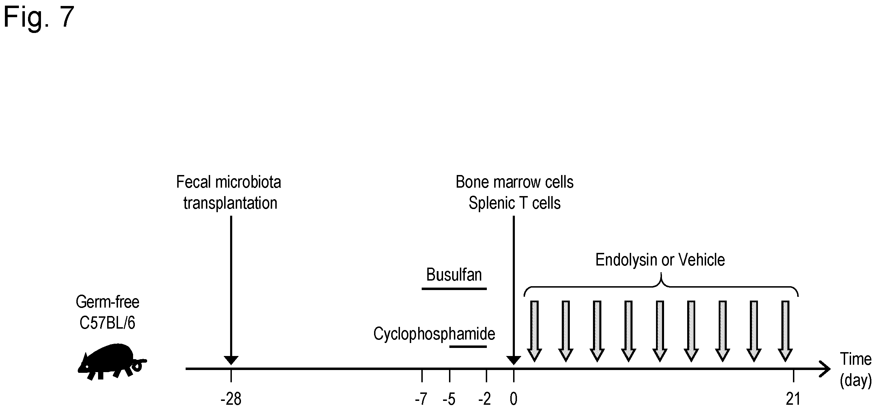

All the animal experiments were performed with the approval of the Animal Care and Use Committee of Osaka Metropolitan University. The overview of the experimental method in the present Example is shown in Figure 7. In this Example, an experiment was performed in the same manner as in Example 5 except that a suspension of 200 µL obtained by diluting the feces (OCU031_14) derived from a patient who exhibited *Enterococcus* dominance 16-fold with an anaerobic culture medium was orally administered. The survival rate after Day 0 was monitored every day, and the composition of the enteric microbes on each of Day 0 and Day 8 was analyzed by 16S rRNA gene sequencing.

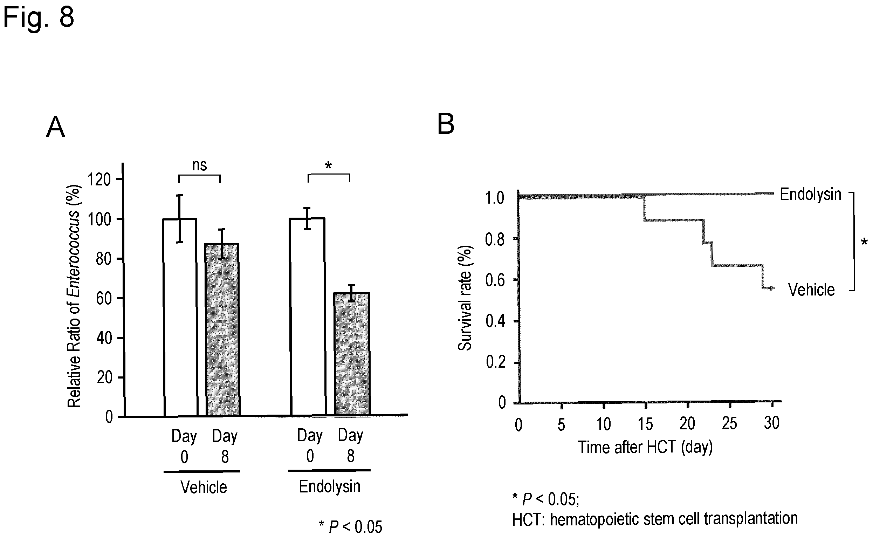

The results are shown in Figure 8. In the GVHD model mice to which the vehicle (SUMO protein having a His tag added thereto) was administered, the ratio of *Enterococcus* bacteria present in the enteric microbes 8 days after the stem cell transplantation did not change significantly. On the other hand, in the GVHD model mice to which the phage-derived endolysin was administered, the ratio of *Enterococcus* bacteria present in the enteric microbes 8 days after the stem cell transplantation decreased significantly (Figure 8A). In addition, 30 days after the stem cell transplantation, nearly half of the GVHD model mice to which the vehicle was administered died, but 100% of the GVHD model mice to which the phage-derived endolysin was administered survived (Figure 8B). Accordingly, it was demonstrated that the administration of the phage-derived endolysin markedly enhances the survival rate.

### <Example 7: Evaluation of lytic activity against further E. faecalis isolates and E. faecium isolates>

### (Purpose)

The lytic activity of the phage-derived endolysin against further *E. faecalis* isolates and *E. faecium* isolates was evaluated. The lytic activity was studied particularly in relation to the presence/absence of cytolysin-related gene and/or the resistance/nonresistance to vancomycin.

### (Methods and Results)

The various species of bacterial strains shown in Table 2 below were obtained from Microbe Division, the BioResource Research Center, RIKEN.

The hemolytic activity of each of the bacterial strains was measured with a hemolysis assay using a Nissui Plate Horse Blood Agar medium. The cytolysin-related gene was detected with PCR. The vancomycin resistance was evaluated using the method described in (2) in Example 2. The vanA gene and the vanB gene that are vancomycin-resistant genes were detected with PCR. Furthermore, each bacterial strain was aerobically cultured in a BHI culture medium, and centrifuged at 300 × g for 5 minutes for collection. The cell pellets were washed, and resuspended in a HiTrap buffer. The phage-derived endolysin was added at a final concentration of 50 µg/mL to the cell resuspension liquid. The resulting suspension was incubated at 37°C, and the lytic activity was measured, using the presence or absence of a bacterial lump as an index.

The results are shown in Table 2 below. The phage-derived endolysin exhibited a lytic activity also against the JCM8902 strain that was a vancomycin-resistant and cytolysin-positive *E. faecalis* isolate. On the other hand, the phage-derived endolysin did not exhibit a lytic activity against the JCM5804 strain that was an *E. faecium* isolate.

**[Table 2]**

| Table 2: Results of various *E. faecalis* isolates and *E. faecium* isolates | | | | | | | |
|---|---|---|---|---|---|---|---|
| Bacterial strains | Bacterial species | Hemolysis assay activity positive: + activity negative: - | Cytolysin positive: + negative: - | Vancomycin-resistance resistant: + nonresistant: - | vanA gene positive: + negative: - | vanB gene positive: + negative: - | Lysis by endolysin lyzed: + not lyzed: - |
| JCM5803 | *E. faecalis* | - | - | - | - | - | + |
| JCM8902 | *E. faecalis* | + | + | + | + | - | + |
| JCM5804 | *E. faecium* | - | - | - | - | - | - |

All publications, patents, and patent applications cited herein are incorporated herein by reference in their entirety.

## Claims

1. A lytic agent for *Enterococcus faecalis*, consisting of a lytic enzyme comprising any of the amino acid sequences of the following (a) to (c) or an active fragment thereof:
(a) the amino acid sequence shown in SEQ ID NO: 1;
(b) an amino acid sequence in which one or multiple amino acids are added, deleted, and/or substituted in the amino acid sequence shown in SEQ ID NO: 1;
(c) an amino acid sequence having 90% or more sequence identity with the amino acid sequence shown in SEQ ID NO: 1.

2. A lytic agent for *Enterococcus faecalis*, comprising a polynucleotide encoding the lytic enzyme or an active fragment thereof of claim 1.

3. The lytic agent of claim 2, wherein said polynucleotide comprises any of the base sequences of the following (a) to (d):
(a) the base sequence shown in SEQ ID NO: 2;
(b) a base sequence in which one or multiple bases are added, deleted, and/or substituted in the base sequence shown in SEQ ID NO: 2;
(c) a base sequence having 90% or more sequence identity with the base sequence shown in SEQ ID NO: 2;
(d) a base sequence which hybridizes with the base sequence complementary to the base sequence shown in SEQ ID NO: 2 under a high-stringent condition.

4. A lytic agent for *Enterococcus faecalis*, comprising an expression vector comprising the polynucleotide of claim 2 or 3.

5. A pharmaceutical composition for treating or preventing an enterococcal infection, comprising the lytic agent of any one of claims 1 to 3, and a pharmacologically acceptable carrier.

6. The pharmaceutical composition of claim 5, wherein said enterococcal infection is intestinal dysbiosis, vancomycin-resistant enterococcus bacteremia, or infective endocarditis.

7. The pharmaceutical composition of claim 6, wherein said intestinal dysbiosis is graft-versus-host disease or alcoholic liver disease.

8. The pharmaceutical composition of claim 7, which is formulated as an enteric-coated preparation.

9. The pharmaceutical composition of claim 8, wherein said carrier is any one or more selected from the group consisting of an excipient, binder, disintegrant, filler, emulsifier, plasticizer, and lubricant.

10. The pharmaceutical composition of claim 5, which is for oral administration, rectal
administration, intraperitoneal administration, or intravenous administration.
